(19) Europäisches Patentamt
European Patent Office
Office européen des brevets

(11) EP 2 154 128 B1

(12) **EUROPÄISCHE PATENTSCHRIFT**

(45) Veröffentlichungstag und Bekanntmachung des Hinweises auf die Patenterteilung:
**29.12.2010 Patentblatt 2010/52**

(51) Int Cl.:
*C07C 269/02* $^{(2006.01)}$ *C07C 271/28* $^{(2006.01)}$
*C07C 323/36* $^{(2006.01)}$

(21) Anmeldenummer: **08014170.8**

(22) Anmeldetag: **08.08.2008**

(54) **Phenylisocyanat-basierte Urethanacrylate mit hohem Brechungsindex**

Phenylisocyanate based urethane acrylates with high refraction index

Uréthane acrylates à base de phénylisocyanate ayant un index de rupture élevé

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(43) Veröffentlichungstag der Anmeldung:
**17.02.2010 Patentblatt 2010/07**

(73) Patentinhaber: **Bayer MaterialScience AG**
**51368 Leverkusen (DE)**

(72) Erfinder:
- **Roelle, Thomas, Dr.**
**51381 Leverkusen (DE)**
- **Bruder, Friedrich-Karl, Dr.**
**47802 Krefeld (DE)**
- **Fäcke, Thomas, Dr.**
**51375 Leverkusen (DE)**
- **Weiser, Marc-Stephan, Dr.**
**51379 Leverkusen (DE)**
- **Hönel, Dennis**
**53909 Zülpich (DE)**

(56) Entgegenhaltungen:
**EP-A- 0 810 210 DE-A1- 2 217 744**

- **E. R. BECKEL, J. NIE, J. W. STANSBURY, C. N. BOWMAN: "Effect of Aryl Substituents on the Reactivity of Phenyl Carbamate Acrylate Monomers" MACROMOLECULES, Bd. 37, Nr. 11, 2004, Seiten 4062-4069, XP002516371**
- **KILAMBI H ET AL: "Influence of molecular dipole on monoacrylate monomer reactivity" POLYMER, ELSEVIER SCIENCE PUBLISHERS B.V, GB, Bd. 46, Nr. 13, 17. Juni 2005 (2005-06-17) , Seiten 4735-4742, XP004904221 ISSN: 0032-3861**
- **E. R. BECKEL, J. W. STANSBURY, C. N. BOWMAN: "Effect of Aliphatic Spacer Substitution on the Reactivity of Phenyl Carbamate Acrylate Monomers" MACROMOLECULES, Bd. 38, 2005, Seiten 3093-3098, XP002555463**

EP 2 154 128 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die Erfindung betrifft neue speziell-substituierte phenylisocyanat-basierte Urethanacrylate mit hohem Brechungsindex sowie ein Verfahren zu deren Herstellung und deren Verwendung.

Beschichtungen mit einem hohen Brechungsindex (n) sind aus verschiedenen Anwendungen bekannt, beispielsweise bei optischen Linsen, Antireflexbeschichtungen, planaren Wellenleitern oder holographisch beschreibbaren Filmen. Beschichtungen mit hohen Brechungsindizes können prinzipiell nach verschiedenen Methoden hergestellt werden. Auf rein physikalischem Weg werden im sogenannten "Sputter-Verfahren" hochbrechende Metalloxide, wie beispielsweise $TiO_2$, $Ta_2O_5$, $CeO_2$, $Y_2O_3$ im Hochvakuum über Plasmaverfahren abgeschieden. Während hierbei problemlos Brechungsindizes von über 2.0 im sichtbaren Wellenlängenbereich erreicht werden können, ist das Verfahren relativ aufwändig und teuer.

Aus EP 0964019 A1 und WO 2004/009659 A1 sind organische Polymere, beispielsweise Schwefel haltige Polymere oder halogenierte Acrylate (Tetrabromphenylacrylat, Polyscience Inc.) bekannt, die inhärent einen höheren Brechungsindex als konventionelle Polymere besitzen und die nach einfachen Methoden aus organischen Lösungen nach konventionellen Beschichtungsverfahren auf Oberflächen appliziert werden können. Allerdings sind hierbei die Brechungsindizes auf Werte bis zu ca. 1.7, gemessen im sichtbaren Wellenlängenbereich, beschränkt.

Eine weitere Verfahrensvariante, die zunehmend an Bedeutung gewinnt, basiert auf Metalloxid-Nanopartikeln, die in organische oder polymere Bindemittelsysteme eingearbeitet werden. Die entsprechenden Nanopartikel-Polymer Hybridrezepturen können in einfacher Weise kostengünstig, beispielsweise mittels Spin Coating, auf verschiedene Substrate appliziert werden. Die erreichbaren Brechungsindizes liegen üblicherweise zwischen den zuerst genannten Sputter-Oberflächen und den Schichten aus hochbrechenden Polymeren. Mit steigenden Nanopartikelgehalten lassen sich steigende Brechungsindizes erreichen. Beispielsweise offenbart US 2002/176169 A1 die Herstellung von Nanopartikel-Acrylat Hybridsystemen, wobei die hochbrechenden Schichten ein Metalloxid, wie beispielsweise Titanoxid, Indiummoxid oder Zinnoxid, sowie ein UV-vernetzbares Bindemittel, beispielsweise auf Acrylat-Basis, in organischem Lösemittel enthalten. Nach Spin Coaten, Abdampfen des Lösemittels und UV-Bestrahlung werden entsprechende Beschichtungen mit einem Realteil n des Brechungsindex von 1.60 bis 1.95, gemessen im sichtbaren Wellenlängenbereich, erhalten.

Neben den physikalischen Eigenschaften sind jedoch auch die Verarbeitbarkeit und Kompatibilität mit anderen Bauteilen wichtig. So spielen organische Materialien, die durch Photopolymerisation, meist als Homo- oder Copolymer hochbrechender Monomere erhalten werden, eine wichtige Rolle, beispielsweise für die Herstellung von optischen Bauteilen wie Linsen, Prismen und optischen Beschichtungen (US 5.916.987) oder für die Erzeugung eines Kontrastes in holographischen Materialien (US 6.780.546). Es besteht für solche und ähnliche Anwendungen Bedarf, den Brechungsindex z.B. durch Zumischen von Komponenten mit hohem Brechungsindex gezielt einstellen und über Bereiche variieren zu können.

Für die vorgenannten Anwendungsgebiete kommen typischerweise Polymerisate olefinisch ungesättigter Verbindungen wie vorzugsweise (Meth)acrylate zum Einsatz. Um einen Brechungsindex von 1,5 und höher zu erreichen, können halogensubstituierte aromatische (Meth)acrylate oder spezielle in US 6.794.471 beschriebene Alkylmethacrylate verwendet werden. Insbesondere letztere sind aufgrund ihrer aufwendigen Herstellung unvorteilhaft.

Die Eignung von substituierten Phenylisocyanat-basierten Urethanacrylaten zur Herstellung entsprechender Polymere wurde von Bowman beschrieben (Polymer 2005, 46, 4735-4742).

Aus WO/2008/125199 sind (Meth)acrylate mit einem Brechnungsindex bei $\lambda$ = 532 nm von mindestens 1.5 bekannt, die sich zur Herstellung von optischen Datenträgern, insbesondere solchen für holographische Speicherverfahren eignen und auf technisch verfügbaren Rohstoffen basieren. In diesem Zusammenhang sind auch Phenylisocyanatbasierte Verbindungen bekannt, wobei diese stets auf unsubstituierten Phenylringen auf der Isocyanatseite basieren.

In Photopolymer-Formulierungen spielen hochbrechende Acrylate als kontrastgebende Komponente (US 6.780.546) eine entscheidende Rolle. Das Interferenzfeld aus Signal- und Referenzlichtstrahl (im einfachsten Fall zwei ebene Wellen) wird durch die lokale Photopolymerisation an Orten hoher Intensität im Interferenzfeld durch die hochbrechenden Acrylate in ein Brechungsindexgitter abgebildet, das alle Information des Signals enthält (das Hologramm). Durch Beleuchtung des Hologramms nur mit dem Referenzlichtstrahl kann dann das Signal wieder rekonstruiert werden. Die Stärke des so rekonstruierten Signals im Verhältnis zur Stärke des eingestrahlten Referenzlichts wird Beugungseffizienz genannt, im folgenden DE wie Diffraction Efficiency. Im einfachsten Fall eines Hologramms, das aus der Überlagerung zweier ebener Wellen entsteht ergibt sich die DE aus dem Quotienten der Intensität des bei der Rekonstruktion abgebeugten Lichtes und der Summe der Intensitäten aus eingestrahltem Referenzlicht und abgebeugtem Licht. Je höher die DE desto effizienter ist ein Hologramm in Bezug auf die notwendige Lichtmenge des Referenzlichtes die notwendig ist um das Signal mit einer festen Helligkeit sichtbar zu machen. Hochbrechende Acrylate sind in der Lage, Brechungsindexgitter mit hoher Amplitude zwischen Bereichen mit niedrigstem Brechungsindex und Bereichen mit höchstem Brechungsindex zu erzeugen und damit in Photopolymer-Formulierungen Hologramme mit hohem DE zu ermöglichen.

Aus E. R. Beckel et al "Effect of Aryl Substituents on the Reactivity of Phenyl Carbamate Acrylate Monomers", Macromolecules, Bd. 37, 2004, S. 4062-4069 sind substituierte Urethanacrylate sowie deren Verwendung als Photopolymere bekannt. Derartige Verbindungen sind auch in der DE 22 17 744 A1 und in der EP-A-0 810 210 beschrieben. E. R. Beckel et al "Effect of Aliphatic Spacer Substitution on the Reactivity of Phenyl Carbamate Acrylate Monomers", Macromolecules, Bd. 38, 2005, S. 3093-3098 offenbart thiol- und schwefelhaltige O-(Meth)acrylate sowie optische Harze und Linsen aus diesem Material.

Es wurde nun überraschenderweise gefunden, dass sich spezielle substituierte phenylisocyanatbasierte Urethanacrylate zu Beschichtungen und Formkörpern mit besonders hohen Brechungsindices in Kombination mit verbesserten DE-Werten aushärten lassen und daher eine besonders gute Eignung als Einsatzmaterial für die Herstellung von hochbrechender Materialien, insbesondere optischen Linsen, Antireflexbeschichtungen, planaren Wellenleitern oder holographisch beschreibbaren Materialien zeigen.

Gegenstand der Erfindung sind daher Urethanacrylate der Formel (I).

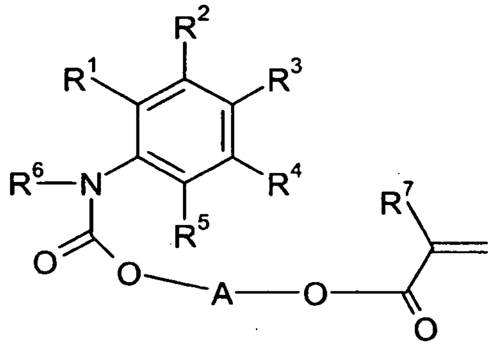

Formel (I)

in der

| | |
|---|---|
| $R^1$, $R^2$, $R^3$, $R^4$, $R^5$ | jeweils für sich ein Wasserstoff- oder Halogenatom oder eine ($C_1$-$C_6$)- Alkyl-, Trifluormethyl-, ($C_1$-$C_6$)-Alkylthio-, ($C_1$-$C_6$)-Alkylseleno-, ($C_1$-$C_6$)- Alkyltelluro- oder Nitro-Gruppe sein können, mit der Maßgabe dass wenigstens ein Substituent der Gruppe $R^1$, $R^2$, $R^3$, $R^4$, $R^5$ nicht Wasserstoff ist |
| $R^6$, $R^7$ | jeweils für sich Wasserstoff oder eine ($C_1$-$C_6$)-Alkylgruppe sein können und |
| A | eine gesättigte oder ungesättigte oder lineare oder verzweigte ($C_1$-$C_6$)-Alkylen-Gruppe oder eine Plyethylenoxide (m = 2-6)- oder Polypropylenoxid(m = 2-6) Gruppe ist, |

wobei auch die entsprechenden Salze, Solvate oder Solvate der Salze der Verbindungen gemäß Formel (I) mit umfasst sind und wenigstens ein Substituent der Gruppe $R^1$, $R^2$, $R^3$, $R^4$ und $R^5$ ein ($C_1$-$C_6$)-Alkylthio-Substistuent ist.

Bevorzugte Reste $R^6$ und $R^7$ sind Wasserstoff-Atome.

Bevorzugt ist der Rest A eine lineare $C_2$-$C_4$ oder verzweigte $C_3$-Alkyl-Gruppe besonders bevorzugt eine linearer $C_2$- oder $C_4$-Alkylen-Gruppe

Die erfmdungswesentlichen Urethanacrylate sind durch Umsetzung von Isocyanaten der Formel (II)

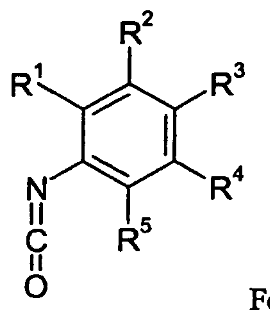

Formel (II)

mit isocyanatreaktiven Verbindungen der Formel (III)

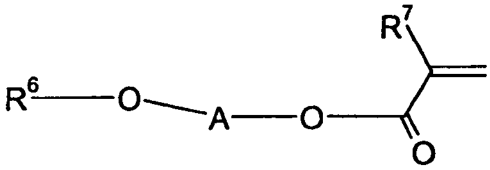

Formel(III)

erhältlich, wobei die Reste die vorgenannte Bedeutung haben.

Als Verbindungen der Formel (ID) können beispielsweise 2-Hydroxyethyl(meth)acrylat, Polyethylenoxid-mono (meth)acrylat, Polypropylenoxidmono(meth)acrylate, Polyalkylenoxidmono(meth)-acrylate, 2-Hydroxypropyl(meth) acrylat, 3-Hydroxypropylacrylat, 4-Hydroxybutyl(meth)acrylat, 3-Hydroxy-2,2-dimethylpropyl(meth)acrylat, Hydroxypropyl(meth)acrylat oder deren Gemische eingesetzt werden.

Bevorzugt sind 2-Hydroxyethylacrylat, 2-Hydroxypropylacrylat, 3-Hydroxypropylacrylat, 4-Hydroxybutylacrylat, Polypropylenoxidmono(meth)acrylate, Polyethylenoxidmono(meth)acrylate oder deren Gemische.

Besonders bevorzugt sind 2-Hydroxyethylacrylat, 2-Hydroxypropylacrylat, 3-Hydroxypropylacrylat und 4-Hydroxybutylacrylat oder deren Gemische.

Bei der Umsetzung von Verbindungen der Formel (II) mit Verbindungen der Formel (III) handelt es sich um eine Urethanisierung. Die Umsetzung von Verbindungen der Formel (II) mit Verbindungen der Formel (III) kann unter zu Hilfenahme der zur Beschleunigung von Isocyanatadditionsreaktionen bekannten Katalysatoren, wie z.B. tertiärer Amine, Zinn-, Zink-, Eisen- oder Bismuthverbindungen, insbesondere Triethylamin, 1,4-Diazabicycl-[2,2,2]-octan, Bismuthoktoat oder Dibutylzinndilaurat erfolgen, die mit vorgelegt oder später zudosiert werden können. Die erfindungsgemäßen Urethanacrylate haben einen Gehalt an Isocyanatgruppen (M = 42) oder freien Restmonomeren von unter 0.5 Gew.-%, bevorzugt unter 0.2 Gew.-%, besonders bevorzugt unter 0.1 Gew.-% bezogen auf das Urethanacrylat. Weiterhin enthalten die erfindungsgemäßen Urethanacrylate Gehalte an nicht umgesetzter Komponente Verbindungen der Formel (III) von unter 1 Gew.-%, bevorzugt unter 0.5 Gew.-% und besonders bevorzugt unter 0.2 Gew.-% bezogen auf das Urethanacrylat. Bei der Herstellung der erfindungsgemäßen Urethanacrylate können die Verbindungen der Formel (II) und die Verbindungen der Formel (III) in einem nicht-reaktiven Lösungsmittel, beispielsweise einem aromatischen oder aliphatischen Kohlenwasserstoff oder einem aromatischen oder aliphatischen halogenierten Kohlenwasserstoff oder einem Lacklösungsmittel wie z.B. Ethylacetat oder Butylacetat oder Aceton oder Butanon oder einem Ether wie Tetrahydrofuran oder tert.-Butylmethylether oder einem dipolar-aprotischen Lösungsmittel wie Dimethylsufoxid oder N- Methylpyrrolidon oder N-Ethylpyrrolidon gelöst werden und in dem Fachmann geläufiger Weise vorgelegt oder zudosiert werden. Nach Reaktionsende werden die nicht-reaktiven Lösungsmittel unter Normaldruck oder unter reduziertem Druck aus dem Gemisch entfernt und der Endpunkt mittel Festgehaltbestimmung ermittelt. Die Festgehalte liegen typischerweise in einem Bereich von 99.999 bis 95.0 Gew.-%, bevorzugt von 99.998 bis 98.0 Gew. -% bezogen auf das Urethanacrylat.

Die erfindungsgemäßen Urethanacrylate können ferner durch den Zusatz von Stabilisatoren gegen ungewollte Polymerisation geschützt werden. Solche Stabilisatoren können sauerstoffhaltiges Gas sein wie auch chemische Stabilisatoren, wie sie beispielsweise in Houben-Weyl, Methoden der organischen Chemie, 4. Auflage, Band XIV/1, Georg Thieme Verlag, Stuttgart 1961, Seite 433 ff. beschrieben sind. Als Beispiele seien genannt: Natriumdithionit, Natriumhydrogensulfid, Schwefel, Hydrazin, Phenylhydrazin, Hydrazobenzol, N-Phenyl-β-naphthylamin, N-Phenyl-ethanoldiamin, Dinitrobenzol, Picrinsäure, p-Nitroso-dimethylanilin, Diphenylnitrosamin, Phenole wie para-Methoxyphenol, 2,5-Di-tert.-Butylhydrochinon, 2,6-Di-tert.-butyl-4-methylphenol, p-tert.-Butyl-brenzcatechin oder 2,5-Di-tert.-amyl-hydrochinon, Tetramethyl-thiuramdisulfid, 2-Mercaptobenzothiazol, Dimethyl-dithiocarbaminsäure-natriumsalz, Phenothiazin, N-Oxyl-Verbindungen wie z.B. 2,2,6,6-Tetramethylpiperidin-N-Oxid (TEMPO) oder eines seiner Derivate. Bevorzugt sind 2,6-Di-tert.-butyl-4-methylphenol und para-Methoxyphenol sowie deren Mischungen. Solche Stabilisatoren werden typischerweise in einer Menge von 0.001 bis 1 Gew.-%, bevorzugt 0.01 bis 0.5 Gew. -% bezogen auf das zu stabilisierende Urethanacrylat eingesetzt.

Schichten, Schichtaufbauten und Formkörper erhältlich aus den erfindungsgemäßen Urethanacrylaten der Formel (I) weisen typischerweise einen Brechungsindex bei 405 nm von >1.50, bevorzugt >1.55, besonders bevorzugt >1.58 auf und sind daher ebenfalls ein Gegenstand der Erfindung.

Schichten, Schichtaufbauten und Formkörper erhältlich aus Formulierungen, die die erfindungsgemäßen Urethanacrylate der Formel (I) enthalten, weisen ferner typischerweise DE-Werte gemessen mittels Zweistrahlinterferenz in Reflektionsanordnung von > 25%, bevorzugt > 30%, besonders bevorzugt > 40%, ganz besonders bevorzugt > 50% auf. Die genaue Methodenbeschreibung ist im Beispielteil der Anmeldung enthalten.

Die erfindungsgemäßen Urethanacrylate der Formel (I) eignen sich daher hervorragend zur Herstellung von holograpischen Medien und holographischen Photopolymer-Filmen.

Ebenfalls ein Gegenstand der Erfindung ist daher ein Verfahren zur Belichtung von holograpischen Medien und holographischen Photopolymer-Filmen, bei dem die erfindungsgemäßen Urethanacrylate, welche in einer Polymermatrix vorliegen, durch elektromagnetische Strahlung selektiv polymerisiert werden.

Derartige holographische Medien eignen sich nach entsprechender holographischer Belichtung zur Herstellung von holographischen optischen Elementen, die z.B. die Funktion einer optischen Linse, eines Spiegels, eines Umlenkspiegels, eines Filters, einer Streuscheibe, eines Beugungselements, eines Lichtleiters, eines Lichtlenkers, einer Projektionsscheibe und/oder einer Maske haben.

Zudem können damit auch holographische Bilder oder Darstellungen hergestellt werden, wie zum Beispiel für persönliche Portraits, biometrische Darstellungen in Sicherheitsdokumenten, oder allgemein von Bilder oder Bildstrukturen für Werbung, Sicherheitslabels, Markenschutz, Markenbranding, Etiketten, Designelementen, Dekorationen, Illustrationen, Sammelkarten, Bilder und dergleichen sowie Bilder, die digitale Daten repräsentieren können, u.a auch in Kombination mit den zuvor dargestellen Produkten.

**<u>Beispiele:</u>**

Sofern nicht abweichend vermerkt beziehen sich alle Prozentangaben auf Gewichtsprozent.

Die Messung des Brechungsindex erfolgte bei einer Wellenlänge von 405 nm. Der Brechungsindex n in Abhängigkeit von der Wellenlänge der Proben wurden aus den Transmissions- und Reflexionsspektren erhalten. Dazu wurden ca. 100 - 300 nm dicke Filme der Proben auf Quarzglasträger aus verdünnter Lösung in Butylacetat aufgeschleudert. Das Transmissions- und Reflexionsspektrum dieses Schichtpaketes wurde mit einem Spektrometer der Firma STEAG ETA-Optik, CD-Measurement System ETA-RT gemessen und danach die Schichtdicke und der spektrale Verlauf von n an die gemessenen Transmissions- und Reflexionsspektren angepasst. Dies geschieht mit der internen Software des Spektrometers und erfordert zusätzlich die n Daten des Quarzglassubstrates, die in einer Blindmessung vorab bestimmt wurden.

**<u>Beispiel 1:</u> 2-({[3-(Methylsulfanyl)phenyl]carbamoyl}oxy)ethylprop-2-enoat**

In einem 100 mL Rundkolben wurden 0.02 g 2,6-Di-tert.-butyl-4-methylphenol, 0.01 g Desmorapid Z, 11.7 g 3-(Methylthio)phenylisocyanat vorgelegt und vorgelegt und auf 60 °C erwärmt. Anschließend wurden 8.2 g 2-Hydroxyethylacrylat zugetropft und die Mischung weiter auf 60 °C gehalten, bis der Isocyanatgehalt unter 0.1 % gesunken war. Danach wurde abgekühlt. Das Produkt wurde als hellgelbe Flüssigkeit erhalten.

**<u>Beispiel 2:</u> 2-({[3-(Methylsulfanyl)phenyl]carbamoyl}oxy)propylprop-2-enoat**

In einem 250 mL Rundkolben wurden 0.05 g 2,6-Di-tert.-butyl-4-methylphenol, 0.02 g Desmorapid Z, 26.8 g 3-(Methylthio)phenylisocyanat in 50 g Ethylacetat vorgelegt und auf 60 °C erwärmt. Anschließend wurden 21.1 g 2-Hydroxypropylacrylat zugetropft und die Mischung weiter auf 60 °C gehalten, bis der Isocyanatgehalt unter 0.1 % gesunken war. Danach wurde das Ethylacetat bei 5 mbar abdestilliert und abgekühlt. Das Produkt wurde als hellgelbe Flüssigkeit erhalten.

**<u>Beispiel 3:</u> 2-({[3-(Methylsulfanyl)phenyl]carbamoyl}oxy)butylprop-2-enoat**

In einem 250 mL Rundkolben wurden 0.05 g 2,6-Di-tert.-butyl-4-methylphenol, 0.02 g Desmorapid Z, 26.7 g 3-(Methylthio)phenylisocyanat vorgelegt und auf 60 °C erwärmt. Anschließend wurden 23.3 g 2-Hydroxybutylacrylat zugetropft und die Mischung weiter auf 60 °C gehalten, bis der Isocyanatgehalt unter 0.1 % gesunken war. Danach wurde das Ethylacetat bei 5 mbar abdestilliert und abgekühlt. Das Produkt wurde als kristalliner Feststoff erhalten.

**<u>Beispiel 4:</u> 2-{2-[2-({[3-(Methylsulfanyl)phenyl]carbamoyl}oxy)ethoxy]ethoxy}ethyl-2-methylprop-2-enoat**

In einem 100 mL Rundkolben wurden 0.02 g 2,6-Di-tert.-butyl-4-methylphenol, 0.01 g Desmorapid Z, 8.6 g 3-(Methylthio)phenylisocyanat vorgelegt und auf 60 °C erwärmt. Anschließend wurden 11.7 g Polyethylenglycol-monomethacrylat (PEM3, Fa. LAPORTE Performance Chemicals UK LTD) zugetropft und die Mischung weiter auf 60 °C gehalten, bis der Isocyanatgehalt unter 0.1 % gesunken war. Danach wurde abgekühlt. Das Produkt wurde als hellgelbe Flüssigkeit erhalten.

**Beispiel 5: 19-{[3-(Methylsulfanyl)phenyl]amino}-19-oxo-3,6,9,12,15,18-hexaoxanonadec-1-ylprop-2-enoat**

In einem 100 mL Rundkolben wurden 0.02 g 2,6-Di-tert.-butyl-4-methylphenol, 0.01 g Desmorapid Z, 6.4 g 3-(Methylthio)phenylisocyanat vorgelegt und auf 60 °C erwärmt. Anschließend wurden 13.6 g Bisomer(TM) PEA 6 (Fa. Cognis Deutschland GmbH & Co KG) zugetropft und die Mischung weiter auf 60 °C gehalten, bis der Isocyanatgehalt unter 0.1 % gesunken war. Danach wurde abgekühlt. Das Produkt wurde als hellgelbe Flüssigkeit erhalten.

**Beispiel 6: 2,5,8,11,14,17-Hexamethyl-19-{[3-(methylsulfanyl)phenyl]amino}-19-oxo-3,6,9,12,15,18-hexaoxanonadec-1-ylprop-2-enoat**

In einem 100 mL Rundkolben wurden 0.02 g 2,6-Di-tert.-butyl-4-methylphenol, 0.01 g Desmorapid Z, 5.6 g 3-(Methylthio)phenylisocyanat vorgelegt und auf 60 °C erwärmt. Anschließend wurden 14.3 g Bisomer(TM) PPA 6 (Fa. Cognis Deutschland GmbH & Co KG) zugetropft und die Mischung weiter auf 60 °C gehalten, bis der Isocyanatgehalt unter 0.1 % gesunken war. Danach wurde abgekühlt. Das Produkt wurde als kristalliner Feststoff erhalten.

**Beispiel 7: 2-({[2-(Methylsulfanyl)phenyl]carbamoyl}oxy)propylprop-2-enoat**

In einem 50 mL Rundkolben wurden 0.008 g 2,6-Di-tert.-butyl-4-methylphenol, 0.004 g Desmorapid Z, 4.8 g 2-(Methylthio)phenylisocyanat in 8.5 g Ethylacetat vorgelegt und auf 60 °C erwärmt. Anschließend wurden 3.7 g 3-Hydroxypropylacrylat zugetropft und die Mischung weiter auf 60 °C gehalten, bis der Isocyanatgehalt unter 0.1 % gesunken war. Danach wurde das Ethylacetat bei 5 mbar abdestilliert und abgekühlt. Das Produkt wurde als hellgelbe Flüssigkeit erhalten.

**Beispiel 8: 2-({[2-(Methylsulfanyl)phenyl]carbamoyl}oxy)butylprop-2-enoat**

In einem 50 mL Rundkolben wurden 0.008 g 2,6-Di-tert.-butyl-4-methylphenol, 0.004 g Desmorapid Z, 4.3 g 2-(Methylthio)phenylisocyanat in 8.5 g Ethylacetat vorgelegt und auf 60 °C erwärmt. Anschließend wurden 4. g 3-Hydroxybutylacrylat zugetropft und die Mischung weiter auf 60 °C gehalten, bis der Isocyanatgehalt unter 0.1 % gesunken war. Danach wurde das Ethylacetat bei 5 mbar abdestilliert und abgekühlt. Das Produkt wurde als hellgelbe Flüssigkeit erhalten.

**Beispiel 9: 2-({[4-(Methylsulfanyl)phenyl]carbamoyl}oxy)ethylprop-2-enoat**

In einem 100 mL Rundkolben wurden 0.02 g 2,6-Di-tert.-butyl-4-methylphenol, 0.01 g Desmorapid Z, 4.7 g 4-(Methylthio)phenylisocyanat in 25 g Ethylacetat vorgelegt und auf 60 °C erwärmt. Anschließend wurden 4.1 g 2-Hydroxyethylacrylat zugetropft und die Mischung weiter auf 60 °C gehalten, bis der Isocyanatgehalt unter 0.1 % gesunken war. Danach wurde das Ethylacetat bei 5 mbar abdestilliert und abgekühlt. Das Produkt wurde als kristalliner Feststoff erhalten.

**Beispiel 10: 2-({[4-(Methylsulfanyl)phenyl]carbamoyl}oxy)propylprop-2-enoat**

In einem 100 mL Rundkolben wurden 0.02 g 2,6-Di-tert.-butyl-4-methylphenol, 0.01 g Desmorapid Z, 14.0 g 4-(Methylthio)phenylisocyanat in 25 g Ethylacetat vorgelegt und auf 60 °C erwärmt. Anschließend wurden 11.0 g 3-Hydroxypropylacrylat zugetropft und die Mischung weiter auf 60 °C gehalten, bis der Isocyanatgehalt unter 0.1 % gesunken war. Danach wurde das Ethylacetat bei 5 mbar abdestilliert und abgekühlt. Das Produkt wurde als hellgelbe Flüssigkeit erhalten.

**Beispiel 11: 2-({[4-(Methylsulfanyl)phenyl]carbamoyl}oxy)butylprop-2-enoat**

In einem 100 mL Rundkolben wurden 0.02 g 2,6-Di-tert.-butyl-4-methylphenol, 0.01 g Desmorapid Z, 13.3 g 4-(Methylthio)phenylisocyanat in 25 g Ethylacetat vorgelegt und auf 60 °C erwärmt. Anschließend wurden 11.6 g 3-Hydroxybutylacrylat zugetropft und die Mischung weiter auf 60 °C gehalten, bis der Isocyanatgehalt unter 0.1 % gesunken war. Danach wurde das Ethylacetat bei 5 mbar abdestilliert und abgekühlt. Das Produkt wurde als kristalliner Feststoff erhalten.

**Vergleichsbeispiel 12: 2-{[(3-Chlorphenyl)carbamoyl]oxy}ethylprop-2-enoat**

In einem 500 mL Rundkolben wurden 0.15 g 2,6-Di-tert.-butyl-4-methylphenol, 0.075 g Desmorapid Z, 85.3 g

3-Chlorphenylisocyanat vorgelegt und auf 60 °C erwärmt. Anschließend wurden 65.5 g 3-Hydroxybutylacrylat zugetropft und die Mischung weiter auf 60 °C gehalten, bis der Isocyanatgehalt unter 0.1 % gesunken war. Danach wurde abgekühlt. Das Produkt wurde als kristalliner Feststoff erhalten.

**Vergleichsbeispiel 13: 2-{[(3-Bromphenyl)carbamoyl]oxy}ethylprop-2-enoat**

In einem 20 mL Probengläschen wurden 0.01 g 2,6-Di-tert.-butyl-4-methylphenol, 0.007 g Desmorapid Z, 9.4 g 3-Bromphenylisocyanat und auf 60 °C erwärmt. Anschließend wurden 5.5 g 3-Hydroxybutylacrylat zugetropft und die Mischung weiter auf 60 °C gehalten, bis der Isocyanatgehalt unter 0.1 % gesunken war. Danach wurde abgekühlt. Das Produkt wurde als kristalliner Feststoff erhalten.

**Vergleichsbeispiel 1: 2-[(Phenylcarbamoyl)oxy]ethylprop-2-enoat**

In einem 500 mL Rundkolben wurden 0.25 g 2,6-Di-tert.-butyl-4-methylphenol, 0.12 g Desmorapid Z, 126.4 g Phenylisocyanat vorgelegt und auf 60 °C erwärmt. Anschließend wurden 123.3 g 2-Hydroxyethylacrylat zugetropft und die Mischung weiter auf 60 °C gehalten, bis der Isocyanatgehalt unter 0.1 % gesunken war. Danach wurde abgekühlt. Das Produkt wurde als kristalliner Feststoff erhalten (Herstellung beschrieben in DE 2329142).

Tabelle 1: Charakterisierung der Beispiele 1-11 und der Vergleichsbeispiele 1, 12 und 13

| Beispiel | Brechungsindex bei $\lambda$ = 405 nm | Doppelbindungsdichte Val/kg (FH) |
|---|---|---|
| 1 | 1.626 | 3.55 |
| 2 | 1.614 | 3.23 |
| 3 | 1.609 | 3.23 |
| 4 | 1.600 | 2.61 |
| 5 | 1.536 | 1.93 |
| 6 | 1.532 | 1.71 |
| 7 | 1.588 | 3.38 |
| 8 | 1.591 | 3.23 |
| 9 | 1.620 | 3.55 |
| 10 | 1.614 | 3.38 |
| 11 | n.b. | 3.23 |
| Vergleichsbeispiel 12 | 1.589 | 3.72 |
| Vergleichsbeispiel 13 | 1.602 | 3.19 |
| **Vergleichsbeispiel 1** | 1.591 | 4.26 |

Zur Prüfung der optischen Eigenschaften wurden wie im Folgenden beschrieben Medien hergestellt und optisch vermessen:

**Herstellung der Polyol-Komponente:**

In einem 1 L Kolben wurden 0.18 g Zinnoktoat, 374.8 g $\varepsilon$-Caprolacton und 374.8 g eines difunktionellen Polytetrahydrofuranpolyetherpolyols (Equivalentgewicht 500 g/Mol OH) vorgelegt und auf 120 °C aufgeheizt und so lange auf dieser Temperatur gehalten, bis der Festgehalt (Anteil der nicht-flüchtigen Bestandteile) bei 99.5 Gew.-% oder darüber lag. Anschließend wurde abgekühlt und das Produkt als wachsiger Feststoff erhalten.

**Medium 1:**

5.927 g der wie oben beschrieben hergestellten Polyol-Komponente wurden mit 2.50 g Urethanacrylat aus Beispiel 1, 0.10 g CGI 909 (Versuchsprodukt der Fa. Ciba Inc, Basel, Schweiz) und 0.010 g Neu Methylenblau bei 60 °C und 3.50 g N-Ethylpyrilidon gemischt so dass eine klare Lösung erhalten wurde. Anschließend wurde auf 30 °C

abgekühlt, 1.098 g Desmodur® XP 2410 (Versuchsprodukt der Bayer MaterialScience AG, Leverkusen, Deutschland, Hexandiisocyanat-basiertes Polyisocyanat, Anteil an Iminooxadiazindion mindestens 30 %, NCO-Gehalt: 23.5 %) zugegeben und erneut gemischt. Schließlich wurden 0.006 g Fomrez UL 28 (Urethanisierungskatalysator, Handelsprodukt der Fa. Momentive Performance Chemicals, Wilton, CT, USA) zugegeben und erneut kurz gemischt. Die erhaltene, flüssige Masse wurde dann auf eine Glasplatte gegeben und dort mit einer zweiten Glasplatte abgedeckt, die durch Abstandshalter auf einen Abstand von 20 μm gehalten wurde. Dieser Probenkörper wurde zunächst 30 Minuten bei Raumtemperatur liegen gelassen und anschließend zwei Stunden bei 50 °C gehärtet.

Die Medien 2-5 wurden in analoger Art und Weise aus den in Tabelle 1 aufgeführten Beispielen hergestellt.

**Vergleichsmedium:**

5.927 g der wie oben beschrieben hergestellten Polyol-Komponente wurden mit 2.50 g 2-[(Phenylcarbamoyl) oxy]ethylprop-2-enoat (Vergleichsbeispiel 1), 0.10 g CGI 909 (Versuchsprodukt der Fa. Ciba Inc., Basel, Schweiz) und 0.010 g Neu Methylenblau bei 60 °C und 3.50 g N-Ethylpyrilidon gemischt so dass eine klare Lösung erhalten wurde. Anschließend wurde auf 30 °C abgekühlt, 1.098 g Desmodur® XP 2410 (Versuchsprodukt der Bayer MaterialScience AG, Leverkusen, Deutschland, Hexandiisocyanat-basiertes Polyisocyanat, Anteil an Iminooxadiazindion mindestens 30 %, NCO-Gehalt: 23.5 %) zugegeben und erneut gemischt. Schließlich wurden 0.006 g Fomrez UL 28 (Urethanisierungskatalysator, Handelsprodukt der Fa. Momentive Performance Chemicals, Wilton, CT, USA) zugegeben und erneut kurz gemischt. Die erhaltene, flüssige Masse wurde dann auf eine Glasplatte gegeben und dort mit einer zweiten Glasplatte abgedeckt, die durch Abstandshalter auf einen Abstand von 20 μm gehalten wurde. Dieser Probenkörper wurde zunächst 30 Minuten bei Raumtemperatur liegen gelassen und anschließend zwei Stunden bei 50 °C gehärtet.

**Messung der holographischen Eigenschaften der Medien mittels Zweistrahlinterferenz in Reflektionsanordnung:**

Die wie beschrieben hergestellten Medien wurden anschließend mittels einer Messanordnung gemäß Figur 1 wie folgt auf ihre holographischen Eigenschaften geprüft:

Der Strahl eines He-Ne Lasers (Emissionswellenlänge 633 nm) wurde mit Hilfe des Raumfilter (SF) und zusammen mit der Kollimationslinse (CL) in einen parallelen homogenen Strahl umgewandelt. Die finalen Querschnitte des Signal und Referenzstrahls werden durch die Irisblenden (I) festgelegt. Der Durchmesser der Irisblendenöffung beträgt 0.4 cm. Die polarisationsabhängigen Strahlteiler (PBS) teilen den Laserstrahl in zwei kohärente gleich polarisierte Strahlen. Über die λ/2 Plättchen wurden die Leistung des Referenzstrahls of 0.5 mW und die Leistung des Signalstrahls auf 0.65 mW eingestellt. Die Leistungen wurden mit den Halbleiterdetektoren (D) bei ausgebauter Probe bestimmt. Der Einfallswinkel (α) des Referenzstrahls beträgt 21.8°, der Einfallswinkel (β) des Signalstrahl beträgt 41.8°. Am Ort der Probe (Medium) erzeugte das Interferenzfeld der zwei überlappenden Strahlen ein Gitter heller und dunkler Streifen die senkrecht zur Winkelhalbierenden der zwei auf die Probe einfallenden Strahlen liegen (Reflexionshologramm). Der Streifenabstand im Medium beträgt ~ 225 nm (Brechungsindex des Mediums zu ~1.49 angenommen).

<u>Bedeutung der Referenzzeichen in Figur 1:</u>

M = Spiegel, S = Verschluss, SF = Raumfilter, CL = Kollimatorlinse, λ/2 = λ/2 Platte, PBS = polarisationsempfindlicher Strahlteiler, D = Detektor, I = Irisblende, α = 21.8°; β = 41.8°.

Es wurden auf folgende Weise Hologramme in das Medium geschrieben:

Beide Shutter (S) sind für die Belichtungszeit t geöffnet.

Danach wurde bei geschlossenen Shuttern (S) dem Medium 5 Minuten Zeit für die Diffusion der noch nicht polymerisierten Schreibmonomere gelassen.

Die geschriebenen Hologramme wurden nun auf folgende Weise ausgelesen. Der Shutter des Signalstrahls blieb geschlossen. Der Shutter des Referenzstrahls war geöffnet. Die Irisblende des Referenzstrahls wurde auf einen Durchmesser < 1 mm geschlossen. Damit erreichte man das für alle Drehwinkel (Ω) des Mediums der Strahl immer vollständig im zuvor geschriebenen Hologramm lag. Der Drehtisch überstrich nun computergesteuert den Winkelbereich von Ω = 0° bis Ω = 20° mit einer Winkelschrittweite von 0.05°. An jedem angefahrenen Winkel Ω wurden die Leistungen des in der nullten Ordnung transmittierten Strahls mittels des entsprechenden Detektors D und die Leistungen des in die erste Ordnung abgebeugten Strahls mittels des Detektors D gemessen. Die Beugungseffizienz ergab sich bei jedem angefahrenen Winkel Ω als der Quotient aus:

Leistung im Detektor des abgebeugten Strahls / (Leistung im Detektor des abgebeugten Strahls + Leistung im

Detektor des transmittierten Strahls)

Die maximale Beugungseffizienz (DE) des Hologramms, also sein Spitzenwert, wurde ermittelt. Eventuell musste dazu die Position des Detektors des abgebeugten Strahls verändert werden, um diesen maximalen Wert zu bestimmen.

Für eine Formulierung wurde diese Prozedur eventuell mehrfach für verschiedene Belichtungszeiten t an verschiedenen Medien wiederholt, um festzustellen bei welcher mittleren Energiedosis des einfallenden Laserstrahls beim Schreiben des Hologramms DE in den Sättigungswert übergeht. Die mittlere Energiedosis E ergibt sich wie folgt aus den Leistungen der zwei Teilstrahlen (0.50 mW und 0.67 mW), der Belichtungszeit t und dem Durchmesser der Irisblende (0.4 cm):

$$E\ (mJ/cm^2) = 2 \cdot [(0.50\ mW + 0.67\ mW) \cdot t\ (s)]/[\pi \cdot 0.4^2\ cm^2]$$

Die Leistungen der Teilstrahlen wurden so angepasst, dass in dem Medium bei den verwendeten Winkeln $\alpha$ und $\beta$, die gleiche Leistungsdichte erreicht wird.

Dabei ergaben sich folgende Messwerte für DE [%] bei der Dosis E [$mJ/cm^2$]:

Tabelle 2: Holographische Bewertung ausgewählter Beispiele

| Medium | Beispiel | Dosis [$mJ/cm^2$] | DE [%] |
|---|---|---|---|
| 1 | 1 | 37 | 68 |
| 2 | 5 | 37 | 26 |
| 3 | 7 | 73 | 26 |
| 4 | 8 | 73 | 57 |
| 5 | Vergleichsbeispiel 13 | 73 | 38 |
| Vergleichsmedium | Vergleichsbeispiel 1 | 73 | 24 |

Die gefundenen Werte für den Dynamischen Bereich (DE) zeigen, dass das im Vergleichsmedium verwendete Urethanacrylat für die Verwendung in holographischen Medien weniger geeignet ist, wohingegen die Urethanacrylate in den Medien 1 bis 5 für die Herstellung holographischer Medien aufgrund des höheren Wertes für DE sehr gut geeignet sind.

## Patentansprüche

1. Urethanacrylate der Formel (I)

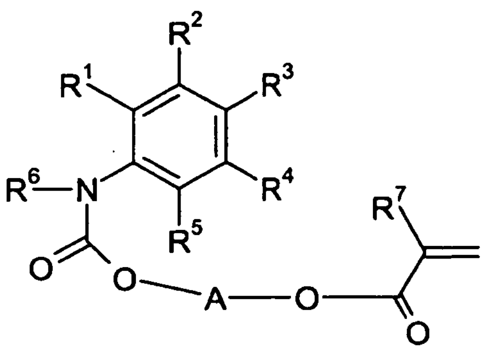

Formel (I)

in der

$R^1$, $R^2$, $R^3$, $R^4$, $R^5$ jeweils für sich ein Wasserstoff- oder Halogenatom oder eine ($C_1$- $C_6$)-Alkyl-, Trifluormethyl-, ($C_1$-$C_6$)-Alkylthio-, ($C_1$-$C_6$)-Alkylseleno-, ($C_1$-$C_6$)- Alkyltelluro- oder Nitro-Gruppe sein können, mit der Maßgabe dass wenigstens ein Substituent der Gruppe $R^1$, $R^2$, $R^3$, $R^4$, $R^5$ nicht Wasserstoff ist
$R^6$, $R^7$ jeweils für sich Wasserstoff oder eine ($C_1$-$C_6$)-Alkylgruppe sein können und
A eine gesättigte oder ungesättigte oder lineare oder verzweigte ($C_1$-$C_6$)-Alkylen-Gruppe oder eine Polyethylenoxid(m = 2-6)- oder Polypropylenoxid(m = 2-6)-Gruppe ist,

wobei auch die entsprechenden Salze, Solvate oder Solvate der Salze der Verbindungen gemäß Formel (I) mit umfasst sind, **dadurch gekennzeichnet, dass** wenigstens ein Substituent der Gruppe $R^1$, $R^2$, $R^3$, $R^4$ und $R^5$ ein ($C_1$-$C_6$)-Alkylthio-Substistuent ist.

**2.** Urethanacrylate gemäß Anspruch 1, **dadurch gekennzeichnet, dass** in Formel (I) $R^6$ und $R^7$ Wasserstoff-Atome sind.

**3.** Urethanacrylate gemäß einem der Ansprüche 1 bis 2, **dadurch gekennzeichnet, dass** in Formel (I) A eine lineare $C_2$-$C_4$ oder verzweigte $C_3$-Alkylengruppe ist.

**4.** Verfahren zur Herstellung von Urethanacrylaten gemäß einem der Ansprüche 1 bis 3, bei dem Isocyanate der Formel (II)

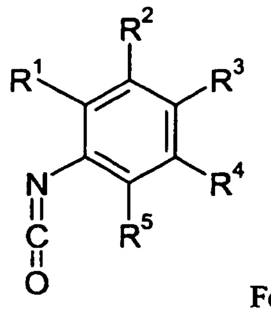

Formel (II)

mit Verbindungen der Formel (III)

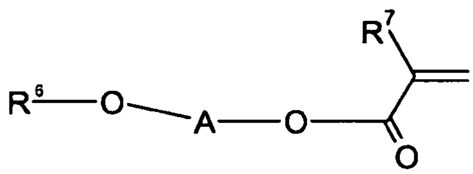

Formel (III)

umgesetzt werden, wobei

$R^1$, $R^2$, $R^3$, $R^4$, $R^5$ jeweils für sich ein Wasserstoff- oder Halogenatom oder eine ($C_1$- $C_6$)-Alkyl-, Trifluormethyl-, ($C_1$-$C_6$)-Alkylthio-, ($C_1$-$C_6$)-Alkylseleno-, ($C_1$-$C_6$)- Alkyltelluro- oder Nitro-Gruppe sein können, mit der Maßgabe dass wenigstens ein Substituent der Gruppe $R^1$, $R^2$, $R^3$, $R^4$, $R^5$ nicht Wasserstoff ist
$R^6$, $R^7$ jeweils für sich Wasserstoff oder eine ($C_1$-$C_6$)-Alkylgruppe sein können und
A eine esättigte oder ungesättigte oder lineare oder verzweigte ($C_1$-$C_6$)-Alkylengruppe oder eine Polyethylenoxid (m = 2-6)- oder Polypropylenoxid(m = 2-6) Gruppe ist,

**dadurch gekennzeichnet, dass** wenigstens ein Substituent der Gruppe $R^1$, $R^2$, $R^3$, $R^4$ und $R^5$ ein ($C_1$-$C_6$)-Alkylthio-Substistuent ist.

**5.** Verfahren gemäß Anspruch 4, **dadurch gekennzeichnet, dass** als Verbindungen der Formel (III) 2-Hydroxyethylacrylat, 3-Hydroxypropylacrylat, 4-Hydroxybutylacrylat, Polypropylenoxidmono(meth)acrylate, Polyethylenoxid-

mono(meth)acrylate oder deren Gemische eingesetzt werden.

6. Verwendung der Urethanacrylate gemäß einem der Ansprüche 1 bis 3 zur Herstellung holograpischer Medien oder holographischer Photopolymer-Filme.

7. Verfahren zur Belichtung von holograpischen Medien und holographischen Photopolymer-Filmen, bei dem Urethanacrylate gemäß einem der Ansprüche 1 bis 3, welche in einer Polymermatrix vorliegen und durch elektromagnetische Strahlung selektiv auspolymerisiert werden.

8. Schichten, Schichtaufbauten oder Formkörper erhältlich unter Verwendung von Urethanacrylaten gemäß einem der Ansprüche 1 bis 3.

9. Schichten, Schichtaufbauten oder Formkörper gemäß Anspruch 8, **dadurch gekennzeichnet, dass** sie einen Brechungsindex bei 405 nm von >1.50 aufweisen.

10. Schichten, Schichtaufbauten oder Formkörper gemäß Anspruch 8 oder 9, **dadurch gekennzeichnet, dass** diese Diffraction Efficiency-Werte gemessen mittels Zweistrahlinterferenz in Reflektionsanordnung von > 25% aufweisen.

11. Schichten, Schichtaufbauten oder Formkörper gemäß einem der Ansprüche 8 bis 10, **dadurch gekennzeichnet, dass** es sich dabei um optischen Linsen, Spiegel, Umlenkspiegel, Filter, Streuscheiben, Beugungselemente, Lichtleiter, Lichtlenker, Projektionsscheiben, Masken, persönliche Portraits, biometrische Darstellungen in Sicherheitsdokumenten, Bilder oder Bildstrukturen handelt.

12. Holographische optische Elemente und holographische Bilder, die durch ein Verfahren nach Anspruch 7 hergestellt werden.

## Claims

1. Urethane acrylates of the Formula (I)

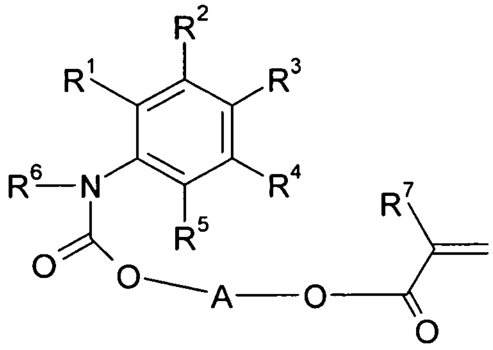

Formula (I)

in which

$R^1$, $R^2$, $R^3$, $R^4$, $R^5$, in each case by themselves, may be a hydrogen or halogen atom or a $(C_1\text{-}C_6)$ - alkyl, trifluoromethyl, $(C_1\text{-}C_6)$-alkylthio, $(C_1\text{-}C_6)$ -alkylseleno, $(C_1\text{-}C_6)$ -alkyltelluro or nitro group, with the proviso that at least one substituent of the group $R^1$, $R^2$, $R^3$, $R^4$, $R^5$ is not hydrogen,
$R^6$, $R^7$, in each case by themselves, may be hydrogen or a $(C_1\text{-}C_6)$ -alkyl group and
A is a saturated or unsaturated or linear or branched $(C_1\text{-}C_6)$ -alkylene group or a polyethylene oxide (m = 2-6) or polypropylene oxide (m = 2-6) group,

the corresponding salts, solvates or solvates of the salts of the compounds according to Formula (I) also being included **characterized in that** at least one substituent of the group $R^1$, $R^2$, $R^3$, $R^4$ and $R^5$ is a $(C_1\text{-}C_6)$-alkylthio substituent.

2. Urethane acrylates according to Claim 1, **characterized in that**, in Formula (I), $R^6$ and $R^7$ are hydrogen atoms.

3. Urethane acrylates according to any of Claims 1 to 2, **characterized in that**, in Formula (I), A is a linear $C_2$-$C_4$ or branched $C_3$-alkylene group.

4. Process for the preparation of urethane acrylates according to any of Claims 1 to 3, in which isocyanates of the Formula (II)

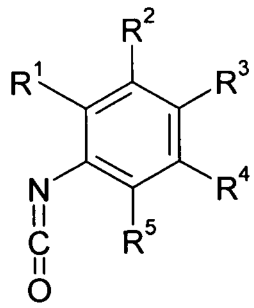

Formula (II)

are reacted with compounds of the Formula (III)

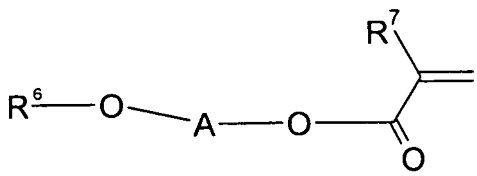

Formula (III)

in which

$R^1$, $R^2$, $R^3$, $R^4$, $R^5$, in each case by themselves, may be a hydrogen or halogen atom or a ($C_1$-$C_6$) - alkyl, trifluoromethyl, ($C_1$-$C_6$)-alkylthio, ($C_1$-$C_6$)-alkylseleno, ($C_1$-$C_6$)-alkyltelluro or nitro group, with the proviso that at least one substituent of the group $R^1$, $R^2$, $R^3$, $R^4$, $R^5$ is not hydrogen,
$R^6$, $R^7$, in each case by themselves, may be hydrogen or a ($C_1$-$C_6$)-alkyl group and
A is a saturated or unsaturated or linear or branched ($C_1$-$C_6$)-alkylene group or a polyethylene oxide (m =2-6) or polypropylene oxide (m = 2-6) group,

**characterized in that** at least one substituent of the group $R^1$, $R^2$, $R^3$, $R^4$ and $R^5$ is a ($C_1$-$C_6$) -alkylthio substituent.

5. Process according to Claim 4, **characterized in that** 2-hydroxyethyl acrylate, 3-hydroxypropyl acrylate, 4-hydroxybutyl acrylate, polypropyleneoxide mono(meth)acrylates, polyethylene oxide mono(meth)acrylates or mixtures thereof are used as compounds of the Formula (III).

6. Use of the urethane acrylates according to any of Claims 1 to 3 for the production of holographic media or holographic photopolymer films.

7. Process for exposing holographic media and holographic photopolymer films to light, in which urethane acrylates according to any of Claims 1 to 3, which are present in a polymer matrix and are selectively polymerized by electromagnetic radiation.

8. Layers, layer structures or mouldings obtainable using urethane acrylates according to any of Claims 1 to 3.

9. Layers, layer structures or mouldings according to Claim 8, **characterized in that** they have a refractive index of >1.50 at 405 nm.

**10.** Layers, layer structures or mouldings according to Claim 8 or 9, **characterized in that** they have diffraction efficiency values, measured by means of two-beam interference in reflection arrangement, of > 25%.

**11.** Layers, layer structures or mouldings according to any of Claims 8 to 10, **characterized in that** they are optical lenses, mirrors, deflection mirrors, filters, diffusion screens, diffraction elements, waveguides, light guides, projection screens, masks, personal portraits, biometric presentations in security documents, images or image structures.

**12.** Holographic optical elements and holographic images which are produced by a process according to Claim 7.

**Revendications**

**1.** Acrylates d'uréthane de formule (I)

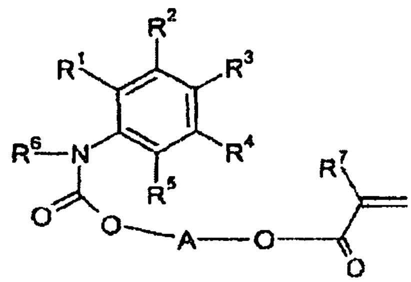

Formule (I)

où

$R^1$, $R^2$, $R^3$, $R^4$, $R^5$ peuvent représenter, à chaque fois en soi, un atome d'hydrogène ou d'halogène ou un groupe ($C_1$-$C_6$)-alkyle, trifluorométhyle, ($C_1$-$C_6$)- alkylthio, ($C_1$-$C_6$)-alkylséléno, ($C_1$-$C_6$)-alkyltelluro ou nitro, à condition qu'au moins un substituant du groupe $R^1$, $R^2$, $R^3$, $R^4$, $R^5$ ne soit pas hydrogène
$R^6$, $R^7$ peuvent représenter à chaque fois en soi hydrogène ou un groupe ($C_1$-$C_6$)-alkyle et
A représente un groupe ($C_1$-$C_6$)-alkylène saturé ou insaturé ou linéaire ou ramifié ou un groupe poly(oxyde d'éthylène) (m = 2-6) ou un groupe poly(oxyde de propylène) (m = 2-6),

où les sels, solvates ou solvates des sels des composés selon la formule (I) sont également inclus, **caractérisés en ce qu'**au moins un substituant du groupe $R^1$, $R^2$, $R^3$, $R^4$ et $R^5$ est un substituant ($C_1$-$C_6$)-alkylthio.

**2.** Acrylates d'uréthane selon la revendication 1, **caractérisés en ce que** dans la formule (I) $R^6$ et $R^7$ représentent des atomes d'hydrogène.

**3.** Acrylates d'uréthane selon l'une quelconque des revendications 1 à 2, **caractérisés en ce que** dans la formule (I) A représente un groupe $C_2$-$C_4$-alkylène linéaire ou un groupe $C_3$-alkylène ramifié.

**4.** Procédé pour la préparation d'acrylates d'uréthane selon l'une quelconque des revendications 1 à 3, dans lequel on transforme des isocyanates de formule (II)

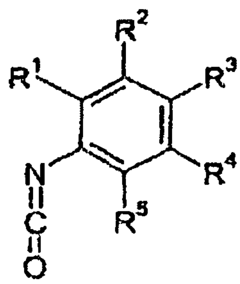

Formule (II)

avec des composés de formule (III)

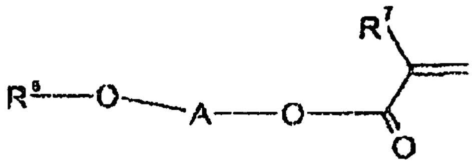

Formule (III)

où

$R^1$, $R^2$, $R^3$, $R^4$, $R^5$ peuvent représenter, à chaque fois en soi, un atome d'hydrogène ou d'halogène ou un groupe ($C_1$-$C_6$)-alkyle, trifluorométhyle, ($C_1$-$C_6$)- alkylthio, ($C_1$-$C_6$)-alkylséléno, ($C_1$-$C_6$)-alkyltelluro ou nitro, à condition qu'au moins un substituant du groupe $R^1$, $R^2$, $R^3$, $R^4$, $R^5$ ne soit pas hydrogène
$R^6$ $R^7$ peuvent représenter à chaque fois en soi hydrogène ou un groupe ($C_1$-$C_6$)-alkyle et
A représente un groupe ($C_1$-$C_6$)-alkylène saturé ou insaturé ou linéaire ou ramifié ou un groupe poly(oxyde d'éthylène) (m = 2-6) ou un groupe poly(oxyde de propylène) (m = 2-6),

**caractérisé en ce qu'**au moins un substituant du groupe $R^1$, $R^2$, $R^3$, $R^4$ et $R^5$ est un substituant ($C_1$-$C_6$)-alkylthio.

5. Procédé selon la revendication 4, **caractérisé en ce qu'**on utilise comme composés de formule (III) l'acrylate de 2-hydroxyéthyle, l'acrylate de 3-hydroxypropyle, l'acrylate de 4-hydroxybutyle, les mono(méth)acrylates de poly (oxyde de propylène), les mono(méth)acrylates de poly(oxyde d'éthylène) ou leurs mélanges.

6. Utilisation des acrylates d'uréthane selon l'une quelconque des revendications 1 à 3 pour la préparation de supports holographiques ou de films photopolymères holographiques.

7. Procédé pour éclairer des supports holographiques et des films holographiques photopolymères, dans lequel des acrylates d'uréthane selon l'une quelconque des revendications 1 à 3 qui se trouvent dans une matrice polymère et sont polymérisés sélectivement par un rayonnement électromagnétique.

8. Couches, constructions à couches ou corps façonnés pouvant être obtenus en utilisant les acrylates d'uréthane selon l'une quelconque des revendications 1 à 3.

9. Couches, constructions à couches ou corps façonnés selon la revendication 8, **caractérisés en ce qu'**ils présentent un indice de diffraction à 405 nm de > 1,50.

10. Couches, constructions à couches ou corps façonnés selon la revendication 8 ou 9, **caractérisés en ce qu'**ils présentent des valeurs d'efficacité de diffraction, mesurées par interférence de deux rayons, dans une disposition de réflexion > 25%.

11. Couches, constructions à couches ou corps façonnés selon l'une quelconque des revendications 8 à 10, **caractérisés en ce qu'**il s'agit de lentilles optiques, de miroirs, de miroirs déflecteurs, de filtres, de disques de dispersion, d'éléments de diffraction, de guides de lumière, de déflecteurs de la lumière, de disques de projection, de masques, de portraits de personnes, de représentations biométriques dans des documents de sécurité, d'images ou de structures d'images.

12. Eléments optiques holographiques et images holographiques, qui sont préparés par un procédé selon la revendication 7.

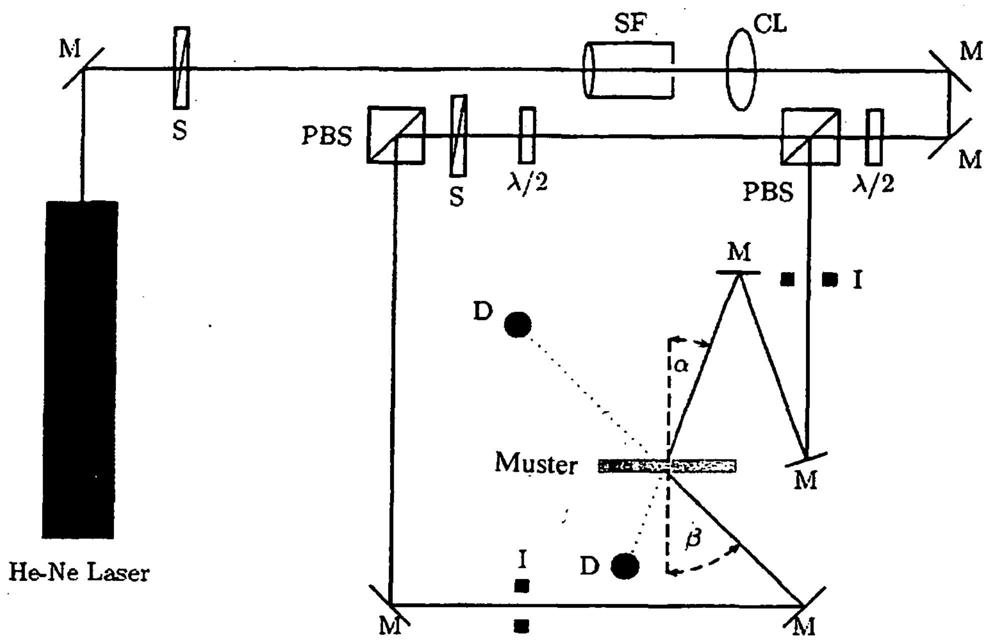


Figur 1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0964019 A1
- WO 2004009659 A1
- US 2002176169 A1
- US 5916987 A
- US 6780546 B
- US 6794471 B
- WO 2008125199 A
- DE 2217744 A1
- EP 0810210 A
- DE 2329142


### In der Beschreibung aufgeführte Nicht-Patentliteratur


- *Polymer,* 2005, vol. 46, 4735-4742
- **E. R. Beckel et al.** Effect of Aryl Substituents on the Reactivity of Phenyl Carbamate Acrylate Monomers. *Macromolecules,* 2004, vol. 37, 4062-4069
- **E. R. Beckel et al.** Effect of Aliphatic Spacer Substitution on the Reactivity of Phenyl Carbamate Acrylate Monomers. *Macromolecules,* 2005, vol. 38, 3093-3098
- **Houben-Weyl.** Methoden der organischen Chemie. Georg Thieme Verlag, 1961, vol. XIV/1, 433 ff